# EUROPÄISCHE PATENTSCHRIFT

(11) **EP 0 523 553 B1**
(45) Veröffentlichungstag und Bekanntmachung des Hinweises auf die Patenterteilung: **03.11.1993**
(21) Anmeldenummer: 92111722.2
(22) Anmeldetag: 09.07.1992
(51) Int. Cl.: C07C 17/00, C07C 19/02

(54) **Verfahren zur Hydrierung von Chlormethanen**
Process for the hydrogenation of chloromethanes
Procédé pour l'hydrogénation des chlorométhanes

(30) Priorität: 15.07.1991 DE 4123396
(43) Veröffentlichungstag der Anmeldung: 20.01.1993
(73) Patentinhaber: WACKER-CHEMIE GMBH, D-81737 München (DE)
(72) Erfinder: Dafinger, Willi, Dr., W-8261 Emmerting (DE); Schmidhammer, Ludwig, Dr., W-8261 Haiming (DE)

(56) Entgegenhaltungen:
- EP-A- 0 432 636
- US-A- 3 579 596

## Beschreibung

Die Erfindung betrifft ein Verfahren zur Hydrierung von Chlormethanen der Formel CHₙClₘ mit n = 0 bis 3 und m = 4-n.

In technischen Verfahren zur Herstellung von Chlorfluormethanen werden überwiegend die entsprechenden Chlormethane als Edukte eingesetzt, in denen dann Chlor stufenweise mit HF durch Fluor ersetzt wird. Vor allem CCl₄ diente bisher als Ausgangsverbindung zur Herstellung von Fluorchlorkohlenwasserstoffen (FCKW's). So gelangt man mit zunehmend schärferen Reaktionsbedingungen bezüglich eingesetzter HF-Menge, Temperatur, Druck, Katalysatortyp und -menge ausgehend von CCl₄ in die Reihe von CFCl₃, CF₂Cl₂ und CF₃Cl.

Das zur Herstellung von FCKW's eingesetzte CCl₄ stammte dabei vorwiegend, sei es als Haupt- oder Nebenprodukt, aus den gängigen Syntheseverfahren zur Herstellung von Chlormethanen. Diese sind die thermische Chlorierung oder katalytische Oxychlorierung von Methan, bei denen alle 4 Chlormethan-Derivate nebeneinander anfallen, das Chlorolyse-Verfahren (chlorierende Spaltung von Propen oder Chlor-haltigen C₁-bis C₃-Rückständen) und die Veresterung von Methanol mit Salzsäure zu Methylchlorid mit nachfolgender Weiterchlorierung von Methylchlorid zu höherchlorierten Chlormethanen.

Bei der stark exothermen, über freie Radikale verlaufenden Gasphasenchlorierung von Methan (400-450°C, geringer Überdruck) entstehen bei äquimolarem CH₄/Cl₂-Verhältnis alle chlorierten Methane nebeneinander: 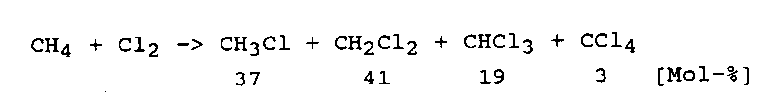
Eine gezielte Höherchlorierung ist dabei durch Rückführung der niederchlorierten Produkte zu erreichen.

Bei der chlorierenden Spaltung von Propen bei Temperaturen von 600 bis 700°C und einem Druck von 2 bis 5 bar entsteht neben Perchlorethylen auch Tetrachlorkohlenstoff:

CH₂=CHCH₃ + Cl₂ → Cl₂C=CCl₂ + CCl₄ + HCl

In Abhängigkeit von den Reaktionsbedingungen und dem Edukt-Verhältnis kann das Mengenverhältnis CCl₄/Per zwischen 65/35 und 35/65 varriieren, das heißt es fallen in jedem Fall erhebliche Mengen an Tetrachlorkohlenstoff an.

Da der Haupteinsatzbereich für CCl₄, die Verwendung als Edukt in der Herstellung von Fluorchlorkohlenwasserstoffen, aufgrund der massiven Produktionsbeschränkungen und des zu erwartenden Produktionsstops in Zukunft wegfallen wird, galt es, neue Einsatzmöglichkeiten für das in den obengenannten Prozessen anfallende CCl₄ aufzudecken.

Eine Möglichkeit besteht in der Verwendung von CCl₄ als Edukt für die Herstellung von Chlormethanen, beispielsweise Chloroform. Unter den Chlormethanen hat vor allem Chloroform eine aktuelle Bedeutung als Edukt für die Herstellung von Polytetrafluoroethylene. Bei der von Chloroform ausgehenden Synthese des Tetrafluoroethylen-Monomeren werden im ersten Schritt zwei Chlor durch Fluor ersetzt:

CHCl₃ + 2 HF -> CHClF₂ + 2 HCl

Durch die nachfolgende thermische Hydrochlorierung von CHClF₂ entsteht monomeres Tetrafluorethylen:

2 CHClF₂ → F₂C=CF₂ + 2 HCl

In der US-A-357 9596 wird ein Verfahren zur Hydrierung von CCl₄ und CHCl₃ beschrieben, wobei die Reaktion in Gegenwart eines Metalls aus der Gruppe Ru, Rh, Pd, Os, Ir, Pt, Cu, Ag und Au katalysiert wird und das Edelmetall für sich oder auf einem Träger aus Tonerde, Aktivhohle oder Kieselgel eingesetzt werden kann.

Es bestand daher die Aufgabe, ein Verfahren zur Herstellung von Chlormethanen, speziell von Chloroform, zu entwickeln, welches von höher-chlorierten Chlormethanen, wie etwa Tetrachlormethan, als Edukt ausgeht.

Gelöst wurde die Aufgabe durch ein Hydrierverfahren, bei dem ein Trägerkatalysator verwendet wird, der als aktive Komponenten Kupfer (elementar oder chemisch gebunden), eine Rhodium/Palladium-Verbindung und eine Phosphorverbindung enthält.

Der erfindungsgemäße Katalysator ist aus der DE-A 3941037 als Katalysator für die Hydrierung von Perchlorethylen zu Trichlorethylen bekannt. Vor diesem technologischen Hintergrund war nicht zu erwarten, daß dieser Katalysator auch bei der Hydrierung von Chlormethanen aktiv ist, da hier ein anderer Reaktionsmechanismus vorliegt und die Bindungskräfte von C₁-Körpern nicht mit denen von C₂-Körpern zu vergleichen sind.

Gegenstand der Erfindung ist ein Verfahren zur Hydrierung von Chlormethanen der Formel CHₙClₘ mit n = 0 bis 3 und m = 4-n mit Wasserstoff bei einer Temperatur von 150 bis 250°C und unter einem Druck von 1 bis 10 bar mittels eines Trägerkatalysators, enthaltend einen Aktivkohleträger mit mehr als 500 m²/g BET-Oberfläche, 0.5 bis 20 Gew.% Kupfer in elementarer oder chemisch gebundener Form und 0.01 bis 1.0 Gew.% Rhodium oder Palladium in elementarer oder chemisch gebundener Form und mit 0.1 bis 10.0 Gew.% eines wasserlöslichen Phosphoniumhalogenids.

Das erfindungsgemäße Verfahren eignet sich vorzugsweise zur Hydrierung von Methylenchlorid, Chloroform und Tetrachlormethan zu den entsprechend niedriger chlorierten Verbindungen. Insbesonders eignet es sich zur Herstellung von Chloroform aus Tetrachlormethan.

Als Katalysatorträger wird Aktivkohle, vorzugsweise in gekörnter Form, eingesetzt. Die Korngröße beträgt in einer bevorzugten Ausführungsform 2 bis 10 mm. Der Aktivkohleträger hat eine BET-Oberfläche von mehr als 500 m²/g, vorzugsweise von 500 bis 1400 m²/g.

Das Kupfer wird zu 0.5 bis 20.0 Gew.%, vorzugsweise zu 5.0 bis 15.0 Gew.%, bezogen auf das Gesamtgewicht aus Katalysatorträger und Aktivkomponenten, in elementarer oder chemisch gebundener Form auf den Träger aufgetragen. Besonders bevorzugt werden wasserlösliche Kupfersalze, insbesonders CuCl₂, eingesetzt.

Rhodium wird zu 0.01 bis 1.0 Gew.%, vorzugsweise zu 0.02 bis 0.2 Gew.%, bezogen auf das Gesamtgewicht aus Katalysatorträger und Aktivkomponenten, in elementarer oder chemisch gebundener Form eingesetzt. Besonders bevorzugt werden wasserlösliche Rhodiumverbindungen, insbesonders Komplexsalze des Rhodium(III)-chlorids. Statt Rhodium kann auch Palladium in elementarer oder chemisch gebundener Form und in den eben für Rhodium angegebenen Mengen eingesetzt werden. Besonders bevorzugt sind auch hier wasserlösliche Palladiumverbindungen, wie etwa PdCl₂.

Der Katalysatorträger ist außerdem noch mit 0.1 bis 10.0 Gew.%, vorzugsweise 3.0 bis 7.0 Gew.%, bezogen auf das Gesamtgewicht aus Katalysatorträger und Aktivkomponenten, eines wasserlöslichen Phosphoniumhalogenids imprägniert. Vorzugsweise werden wasserlösliche Phosphoniumhalogenide der allgemeinen Formel (Ph₃PR)X eingesetzt; wobei Ph für einen Phenylrest steht.

R steht für Wasserstoff oder einen, gegebenenfalls substituierten, Alkyl- oder Arylrest.
Beispiele hierfür sind Methyl-, Ethyl-, Propyl-, n-Butyl-, iso-Butyl-, Pentyl-, Hexyl-, Heptyl-, Octyl-, Nonyl-, Benzyl-, p-Chlorbenzyl-, p-tert.-Butylbenzyl-, Allyl-, 2-Methallyl, Chlormethyl-, Dichlormethyl-, Iodmethyl-, Ethoxycarbonylmethyl- oder Acetonyl-Reste.

Als Halogenid X wird Chlorid, Iodid oder Bromid bevorzugt eingesetzt.

Bevorzugte Phosphoniumhalogenide sind Methyltriphenylphosphoniumchlorid, Methyltriphenylphosphoniumbromid, Ethyltriphenylphosphoniumchlorid, Ethyltriphenylphosphoniumbromid, n-Propyltriphenylphosphoniumchlorid, n-Propyltriphenylphosphoniumbromid, Allyltriphenylphosphoniumchlorid, Allyltriphenylphosphoniumbromid, n-Butyltriphenylphosphoniumchlorid und n-Butyltriphenylphosphoniumbromid.

Besonders bevorzugt sind Methyltriphenylphosphoniumchlorid, Methyltriphenylphosphoniumbromid, Ethyltriphenylphosphoniumchlorid und Ethyltriphenylphosphoniumbromid.

Die Herstellung der Phosphoniumhalogenide kann in an sich bekannter Art und Weise erfolgen und ist beispielsweise in der DE-A 3941037 beschrieben.

Zur Imprägnierung werden die einzelnen Komponenten, Kupfer(salz), Rhodium(salz) oder Palladium(salz), und die Phosphoniumverbindung, vorzugsweise in wäßriger Lösung, getrennt oder im Gemisch, auf die Aktivkohle, zum Beispiel durch Tränken, aufgetragen. Anschließend wird der so imprägnierte Katalysatorträger getrocknet.

Zur Umsetzung der Chlormethane wird der Katalysator in geschütteter Form in ein Reaktionsrohr gebracht. Die Reaktion wird bei einer Temperatur von 150 bis 250°C und unter einem Druck von 1 bis 10 bar absolut durchgeführt. Die Chlormethanverbindung wird vorzugsweise in Mengen von 0.5 bis 5.0 Mol pro Stunde und pro Liter an Kontaktmasse (Katalysatorvolumen) zusammen mit der 0.1- bis 2-fachen molaren Menge pro Stunde an Wasserstoff zur Reaktion gebracht.

Die folgenden Beispiele dienen zur weiteren Erläuterung der Erfindung:

### Beispiel 1:

Gekörnte Aktivkohle mit einer BET-Oberfläche von 800 m²/g und einer Korngröße von 3 mm (Degusorb WS IV Spezial, Fa. Degussa) wurde mit wäßrigen Lösungen von CuCl₂, Na₃RhCl₆ und [(C₆H₅)₃PCH₃]Cl getränkt und anschließend getrocknet, so daß der Cu-Gehalt der Aktivkohle 10 Gew.%, der Rh-Gehalt 0.044 Gew.% und der [(C₆H₅)PCH₃]Cl-Gehalt 6.5 Gew.% betragen hat. Die Tetrachlormethanhydrierung wurde in einem Einrohrreaktor mit einem Katalysatorvolumen von 450 ml durchgeführt. Der Reaktor wurde mit 64 g/h (0.4 mol/h) Tetrachlormethan und 9.1 l/h (0.45 mol/h) Wasserstoff belastet. Der Reaktionsdruck betrug 4 bar absolut. Bei einer Reaktionstemperatur von 200°C wurde ein Produktgemisch aus 46.8 Gew% CCl₄, 52.5 Gew% CHCl₃, 0.602 Gew% CH₂Cl₂ und 0.009 Gew% CH₃Cl erhalten.

### Beispiel 2:

Es wurde analog Beispiel 1 vorgegangen. Als Phosphoniumhalogenid wurde allerdings Methyltriphenylphosphoniumbromid eingesetzt. Die Umsatzrate betrug 45 Gew`% CHCl₃.

### Beispiel 3:

Es wurde der in Beispiel 1 hergestellte Katalysator verwendet. Der Einrohrreaktor wurde mit einem Katalysatorvolumen von 450 ml gefüllt und mit 47 g/h (0.4 mol/h) CHCl₃ und 9.1 l/h (0.45 mol/h) H₂ beladen. Der Reaktionsdruck betrug 4 bar absolut. Bei einer Reaktionstemperatur von 200°C wurden nach der Umsetzung 83.43 Gew% CHCl₃, 14.7 Gew% CH₂Cl₂ und als Rest CH₃Cl und Methan erhalten.

## Patentansprüche

1. Verfahren zur Hydrierung von Chlormethanen der Formel CHₙClₘ mit n = 0 bis 3 und m = 4-n mit Wasserstoff bei einer Temperatur von 150 bis 250°C und unter einem Druck von 1 bis 10 bar absolut mittels eines Trägerkatalysators, enthaltend einen Aktivkohleträger mit mehr als 500 m²/g BET-Oberfläche, 0.5 bis 20 Gew.% Kupfer in elementarer oder chemisch gebundener Form und 0.01 bis 1.0 Gew.% Rhodium oder Palladium in elementarer oder chemisch gebundener Form und mit 0.1 bis 10.0 Gew.% eines wasserlöslichen Phosphoniumhalogenids.

2. Verfahren nach Anspruch 1, daurch gekennzeichnet, daß Aktivkohle in gekörnter Form mit einer Korngröße von 2 bis 10 mm und einer BET-Oberfläche von 500 bis 1400 m²/g eingesetzt wird.

3. Verfahren nach Anspruch 1 oder 2, dadurch gekennzeichnet, daß 5.0 bis 15.0 Gew.%, bezogen auf das Gesamtgewicht aus Katalysatorträger und Aktivkomponenten, eines wasserlöslichen Kupfersalzes eingesetzt wird.

4. Verfahren nach Anspruch 3, dadurch gekennzeichnet, daß CuCl₂ eingesetzt wird.

5. Verfahren nach Anspruch 1, 2, 3 oder 4, dadurch gekennzeichnet, daß 0.02 bis 0.2 Gew.%, bezogen auf das Gesamtgewicht aus Katalysatorträger und Aktivkomponenten, einer wasserlöslichen Rhodiumverbindung oder einer wasserlöslichen Palladiumverbindung eingesetzt werden.

6. Verfahren nach Anspruch 5, dadurch gekennzeichnet, daß Rhodium(III)-chlorid oder Palladium(II)-chlorid eingesetzt wird.

7. Verfahren nach einem der Ansprüche 1 bis 6, dadurch gekennzeichnet, daß eine Phosphoniumverbindung der allgemeinen Formel (Ph₃PR)X eingesetzt wird, wobei Ph für einen Phenylrest steht, R für Wasserstoff oder einen substituierten oder unsubstituierten Alkyl- oder Arylrest und X für Chlorid, Bromid oder Iodid steht.

8. Verfahren nach Anspruch 7, dadurch gekennzeichnet, daß als wasserlösliches Phosphoniumhalogenid Methyltriphenylphosphoniumchlorid, Methyltriphenylphosphoniumbromid, Ethyltriphenylphosphoniumchlorid oder Ethyltriphenylphosphoniumbromid eingesetzt wird.

9. Verfahren nach einem der Ansprüche 1 bis 8, dadurch gekennzeichnet, daß die Chlormethanverbindung in Mengen von 0.5 bis 5.0 Mol pro Stunde und pro Liter an Katalysatorvolumen zusammen mit der 0.1- bis 2-fachen molaren Menge pro Stunde an Wasserstoff zur Reaktion gebracht wird.

10. Verfahren nach einem der Ansprüche 1 bis 9, dadurch gekennzeichnet, daß Tetrachlormethan hydriert wird.

## Claims

1. Process for the hydrogenation of chloromethanes of the formula CHₙClₘ, where n = 0 to 3 and m = 4-n, with hydrogen at a temperature of 150 to 250°C and under an absolute pressure of 1 to 10 bar, by means of a supported catalyst comprising an activated charcoal support with a BET surface area of more than 500 m²/g, 0.5 to 20% by weight of copper in elemental or chemically bonded form and 0.01 to 1.0% by weight of rhodium or palladium in elemental or chemically bonded form, and with 0.1 to 10.0% by weight of a water-soluble phosphonium halide.

2. Process according to Claim 1, characterised in that the activated charcoal used is in granular form with a grain size of 2 to 10 mm and a BET surface area of 500 to 1400 m²/g.

3. Process according to Claim 1 or 2, characterised in that 5.0 to 15.0% by weight, based on the total weight of the catalyst support and active components, of a water-soluble copper salt is used.

4. Process according to Claim 3, characterised in that CuCl₂ is used.

5. Process according to any of Claims 1, 2, 3 or 4, characterised in that 0.02 to 0.2% by weight, based on the total weight of the catalyst support and active components, of a water-soluble rhodium compound or a water-soluble palladium compound is used.

6. Process according to Claim 5, characterised in that rhodium(III) chloride or palladium(II) chloride is used.

7. Process according to any of Claims 1 to 6, characterised in that a phosphonium compound of the general formula (Ph₃PR)X is used, Ph being a phenyl radical, R being hydrogen or a substituted or unsubstituted alkyl or aryl radical and X being chloride, bromide or iodide.

8. Process according to Claim 7, characterised in that the water-soluble phosphonium halide used is methyltriphenyl phosphonium chloride, methyltriphenylphosphonium bromide, ethyltriphenylphosphonium chloride or ethyltriphenylphosphonium bromide.

9. Process according to any of Claims 1 to 8, characterised in that the chloromethane compound is reacted in amounts of 0.5 to 5.0 mol per hour and per litre of catalyst volume, together with the 0.1-fold to 2-fold molar amount per hour of hydrogen.

10. Process according to any of Claims 1 to 9, characterised in that carbon tetrachloride is hydrogenated.

## Revendications

1. Procédé d' hydrogénation de chlorométhanes de formule CHₙClₘ dans laquelle n = 0 à 3 et m = 4-n, avec de l'hydrogène, à une température de 150 à 250°C et sous une pression de 1 à 10 bars absolus, au moyen d'un catalyseur avec support, comportant un support en charbon actif avec une surface BET supérieure à 500 m²/g, de 0,5 à 20% en poids de cuivre sous forme élémentaire ou liée chimiquement et de 0,01 à 1,0% en poids de rhodium ou de palladium sous forme élémentaire ou liée chimiquement, et avec de 0,1 à 10,0% en poids d'un halogénure de phosphonium soluble dans l'eau.

2. Procédé selon la revendication 1, caractérisé en ce que le charbon actif est utilisé sous une forme granulée, avec une grosseur des grains de 2 à 10 mm et une surface BET de 500 à 1400 m²/g.

3. Procédé selon la revendication 1 ou 2, caractérisé en ce que l'on utilise de 5,0 à 15,0% en poids, par rapport au poids total de support de catalyseur et de composants actifs, d'un sel de cuivre soluble dans l'eau.

4. Procédé selon la revendication 3, caractérisé en ce que l'on utilise CuCl₂.

5. Procédé selon la revendication 1, 2, 3 ou 4, caractérisé en ce que l'on utilise de 0,02 à 0,2% en poids, par rapport au poids total de support de catalyseur et de composants actifs, d'un composé de rhodium soluble dans l'eau ou d'un composé de palladium soluble dans l'eau.

6. Procédé selon la revendication 5, caractérisé en ce que l'on utilise le chlorure de rhodium (III) ou le chlorure de palladium (II).

7. Procédé selon l'une des revendications 1 à 6, caractérisé en ce que l'on utilise un composé de type phosphonium de formule générale (Ph₃PR)X, où Ph représente un reste phényle, R représente l'hydrogène ou un reste alkyle ou aryle substitué ou non substitué, et X représente le chlorure, le bromure ou l'iodure.

8. Procédé selon la revendication 7, caractérisé en ce que l'on utilise comme halogénure de phosphonium soluble dans l'eau le chlorure de méthyltriphénylphosphonium, le bromure de méthyltriphénylphosphonium, le chlorure d'éthyltriphénylphosphonium ou le bromure d'éthyltriphénylphosphonium.

9. Procédé selon l'une des revendications 1 à 8, caractérisé en ce que l'on fait réagir le composé de type chlorométhane en quantité de 0,5 à 5,0 moles par heure et par litre de volume de catalyseur, avec une quantité de 0,1 à 2 fois molaire, par heure, d'hydrogène.

10. Procédé selon l'une des réactions 1 à 9, caractérisé en ce que l'on hydrogène le tétrachlorométhane.
